# EUROPEAN PATENT APPLICATION

(11) **EP 4 778 707 A1**
(43) Date of publication of application: **22.07.2026**
(21) Application number: 25152765.1
(22) Date of filing: 20.01.2025
(51) Int. Cl.: B29C 65/18, A61F 13/15, B29L 31/48

(54) **ARRANGEMENT AND METHOD FOR PRODUCTION OF WASHABLE REUSABLE HYGIENE PRODUCTS**

(71) Applicant: Spacerpad AB, 435 39 Mölnlycke (SE)
(72) Inventor: KARLANDER, Sebastian, 518 31 Sandared (SE); HÖGBERG, Karin, 435 39 Mölnlycke (SE)
(74) Representative: Lind Edlund Kenamets Intellectual Property AB

(57) **Abstract**

An arrangement for producing a reusable hygiene product comprising a baseplate (5) and at least one mold (7) comprising a central indentation (42) facing the baseplate (5) and a substantially flat section (44) surrounding the indentation (42). The baseplate (5) and at least one mold (7) are configured to be heated to an elevated temperature, and are displaceable to a position in which the mold (7) is in proximity with the base plate (5). The arrangement further comprises a tray (3) comprising at least one compartment (24) having a heat transmissive floor (25) configured to support a stack (30) of layers. The tray (3) can be inserted between the mold (7) and the baseplate (5), allowing the stack (30) to be heat sealed.

## Description

### Field of the invention

The present inventive concept relates to an arrangement and method for producing a washable reusable hygiene product.

### Background of the invention

Throughout the world, and especially in developing nations, there exists a need for washable reusable hygiene products, both from an environmental and an economic standpoint. Most, if not all, current washable reusable sanitary products are sewn by sewing machine. This production method leads to low efficiency of production, and a higher product cost. It is necessary to make washable reusable hygiene products relatively inexpensive to manufacture, also on a small scale, for them to be available to citizens in developing nations.

One washable reusable hygiene product is disclosed in the patent application WO2021/224482, which materials are optimized for the purpose of being easy to wash and dry and inexpensive enough, allowing it to satisfy the need presented above. The washable reusable hygiene product disclosed in WO2021/224482 comprises a liquid retaining spacer material in between a liquid impermeable backing layer and a liquid impermeable annual liner which are heat sealed. This structure allows the washable reusable hygiene product to retain liquid, at the same time as it is washable.

Typically, disposable hygiene products, such as sanitary pads, are mass-produced with large machines in a factory process. The large machines enable cheap and efficient mass production suitable for single-use articles.

However, such production systems require significant investment, and are not economically feasible for low-cost and small scale production of reusable hygiene products.

Document WO2015/079374 discloses a machine more suitable for small-scale and low cost manufacture of disposable multi-layer absorbent articles. However, the machine in WO2015/079374 is primarily intended for absorbent articles, and is not necessarily appropriate for manufacture of hygiene products comprising a liquid retaining spacer material. Also, the machine disclosed in WO2015/079374 is difficult to use, leading to low production efficiency.

### Summary of the invention

It is an object of the current invention to eliminate or at least mitigate the above problem of inefficiency associated with low-cost production of washable reusable hygiene products.

According to a first aspect of the invention, this and other objects are achieved by an arrangement for producing a washable reusable hygiene product including of a stack of layers comprising a liquid impermeable back sheet, a liquid retaining pad, and a heat sealable liquid impermeable annular liner. The arrangement comprises a baseplate, and at least one mold comprising a central indentation facing the baseplate and a substantially flat section surrounding the indentation, wherein the baseplate and at least one mold are configured to be heated to an elevated temperature, and are displaceably arranged between a first position in which a space is formed between the at least one mold and the baseplate and a second position in which the at least one mold is in proximity with the base plate. The arrangement further comprises a tray arranged to be inserted in said space, wherein the tray comprises at least one compartment having a heat transmissive floor configured to support a stack of layers of the product arranged in the compartment, so that, when the tray is inserted into said space and the mold and baseplate are heated to the elevated temperature and displaced to the second position, each mold is received by and is aligned with a respective compartment, thereby compressing a stack of layers in the compartment and heat sealing the annual liner with the back sheet.

The tray makes it possible to arrange the layers of the reusable hygiene product in a stack when the tray is removed from the baseplate and molds, thereby avoiding reaching in between the hot plates. The compartments in the tray will ensure that the stacks align with the molds, even further facilitating the arrangement process.

The heat transmissive floor of the compartment allows heat from the baseplate to reach the stack, so that heat is applied to the stack from both sides when the layers are heat sealed. Applying heat from both sides is important in order to ensure that the annular liner and the liquid retaining pad are both satisfactorily heat sealed to the back sheet.

By having two (or more) trays, one batch of stacks can be arranged in one tray while the heat press is heat sealing another batch of products. This allows for sequential production and thus increased efficiency.

In some embodiments, each compartment has a shape which snugly matches a respective mold, so that pressure can be applied in the entire compartment. This makes it possible to have layers in the stack which completely fill the compartment and thereby can be aligned by the compartment walls.

To further ensure consistent placing of the stack the arrangement may comprise a compartment limiter. The limiter is configured to be inserted into a compartment, wherein the compartment limiter comprises a hole for receiving and ensuring proper placement in the compartment of a liquid retaining pad of the product the arrangement is intended to produce. The compartment limiter's configuration and shape enable placement of the liquid retaining pad in the same place in the compartment each time.

The performance of the reusable hygiene product will be better if the liquid retaining pad has not been too compressed. To achieve a different amount of pressure on the liquid retaining pad, the mold pressing down on the stack of layers has an indentation, where the part of the stack that lines up with the indentation receives less pressure. The indentation in the mold may therefore have a shape which follows the shape of the liquid retaining pad of the product the arrangement is intended to produce, given that the liquid retaining pad is the part of the stack that benefits from a lower pressure being applied to it.

Efficiency may be further increased by adding more compartments and molds to allow for more products to be produced at once. Because of this, there may be two, three, four or more molds, with a corresponding number of compartments in the tray.

In some embodiments, the arrangement is manually operated. The tray may then comprise a grippable portion protruding out of the space between the at least one mold and the baseplate. The tray is best removed from the space to retrieve a produced batch of products and inserted with stacks in the compartments to produce a new batch of products. These actions necessitate the tray being handled. The grippable portion allows handling the tray with a greatly reduced risk of burning one's hands on the baseplate and/or molds. This means that the baseplate and mold can be kept at the elevated temperature between uses, both allowing more consistent results from a consistent temperature than if the temperature had to vary and making the arrangement more energy efficient as the baseplate and molds do not require cooling and reheating between each use.

The elevated temperature may be between 130 and 170 degrees Celsius, preferably between 140 and 160 degrees Celsius. This ensures that the heat sealable liner's heat-sealing is activated without scorching the product. The temperature used of course depends on the material that the heat sealable layers are made of, as the skilled craftsperson of course will account for.

The tray may comprise a PTFE film which is attached to one side of the tray to form the heat transmissive floor of the compartments. The floor of the compartments, being of this material ensures its heat transmissive property. This material is also resistant to sticking to the product after heat pressing, allowing products to be retrieved from the compartments easily.

In some embodiments, inward-facing walls of the compartments may be covered substantially by PTFE tape as it is resistant to sticking to the product after heat pressing, allowing products to be retrieved from the compartments easily.

The relative displacement of mold(s) and base plate can in principle be done manually, by means of an appropriate handle or lever. In a more automated embodiment, the arrangement may comprise an actuator which causes the displacement of the at least one mold and baseplate between the first and second position. This displacement could take the form of the actuator pushing a top plate to which the mold is affixed towards the baseplate. When the displacement takes this form, the actuator may be a hydraulic press. To be able to perform the function of displacing the molds and the baseplate in relation to each other, the actuator may be connected to a frame which is stationary in relation to the baseplate, and move the top plate in relation to the baseplate.

In some embodiments, the liquid retaining pad is made from one or several patches of spacer material. By "spacer material" is here intended a three-dimensional fabric made of an upper layer, a lower layer, and an interconnecting layer of pile filaments that serve as spacer yarns. One example of such a spacer material is the material defined in WO2014/102072, herewith incorporated by reference. Such material has proven to be particularly useful for reusable hygiene products.

The arrangement may comprise a set of guides that ensure that the tray is inserted into a position where each mold is receivable by, and is aligned with, one respective compartment. The consistent inserting position is partly ensured by a pair of guides on either side of the inserting position that force an insertion to be made at a specific spot. The set of guides may further comprise an abutment situated at a depth into the space to which the tray should be inserted. By inserting the tray between the pair of guides until the tray hits the abutment, the tray will be inserted into the same position every time, namely the position where each mold is receivable by and is aligned with one compartment each, ensuring proper operation of the arrangement. The skilled craftsperson will of course realize that other means by which a specific insertion position is ensured are of equal viability to the set of guides described here. If a set of guides is used, the important part is that they ensure a specific positioning of the tray, with the form they take being of no particular importance.

According to a second aspect of the invention, this and other objects are achieved by a method for producing a washable reusable hygiene product comprising the steps: providing a tray with a compartment and a heat transmissive floor, arranging a stack of layers in the compartment. The stack includes a liquid impermeable annular liner, a liquid retaining pad, and a liquid impermeable back sheet. The method further comprising the steps of: inserting the tray into a space between a heated baseplate and a heated mold, displacing the mold in relation to the baseplate so that they press the stack of layers from opposite directions, whereby the stack of layers is heat sealed into an assembled stack, retrieving the assembled stack from the compartment.

This second aspect of the invention provides essentially similar advantages as those associated with the first aspect.

If the arrangement comprises a compartment limiter, the assembling step may further comprise the steps of arranging a compartment limiter to the compartment before adding the liquid retaining pad, removing the compartment limiter after arranging the liquid retaining pad, so that the compartment limiter is able to guide the placement of the liquid retaining pad. This is done to achieve the advantage of using a compartment limiter presented above.

The liquid retaining pad may comprise at least three liquid retaining patches, and may then comprises a smaller patch facing the back sheet, a larger patch facing the liner, and at least one intermediate patch, sandwiched between the smaller and larger patches, the intermediate patch being larger than the smaller patch. The intermediate patch should extend outside the contour of the smaller patch, such that it is brought into contact with the back sheet along its edges. This ensures that also the intermediate patch will become attached to the back sheet, thereby holding it in the intended place and orientation in the finished product.

### Brief description of the drawings

The present invention will be described in more detail with reference to the appended drawings, showing currently preferred embodiments of the invention.
Figure 1 illustrates an arrangement for producing a washable sanitary product according to an embodiment of the present invention.
Figure 2 illustrates the arrangement in figure 1, with the tray inserted.
Figure 3 illustrates an exploded view of the heat press in figure 1-2.
Figure 4 illustrates the tray with four compartments, each compartment showing a different stage of arranging the stack of layers.
Figure 5a is a cross-section view of a mold being received by a compartment, wherein a stack of layers has been placed in the compartment.
Figure 5b is the cross section in figure 5a, but where the stack is compressed into an assembled stack.
Figure 6 illustrates the tray with assembled stacks in the compartments.
Figure 7 illustrates an assembled stack with a dotted line indicating where the stack is to be cut to form it into a finished product.
Figure 8 is a flowchart of a method according to an embodiment of the invention.
Figure 9 illustrates a carrier plate with three molds, according to a further embodiment of the invention.

### Detailed description of preferred embodiments

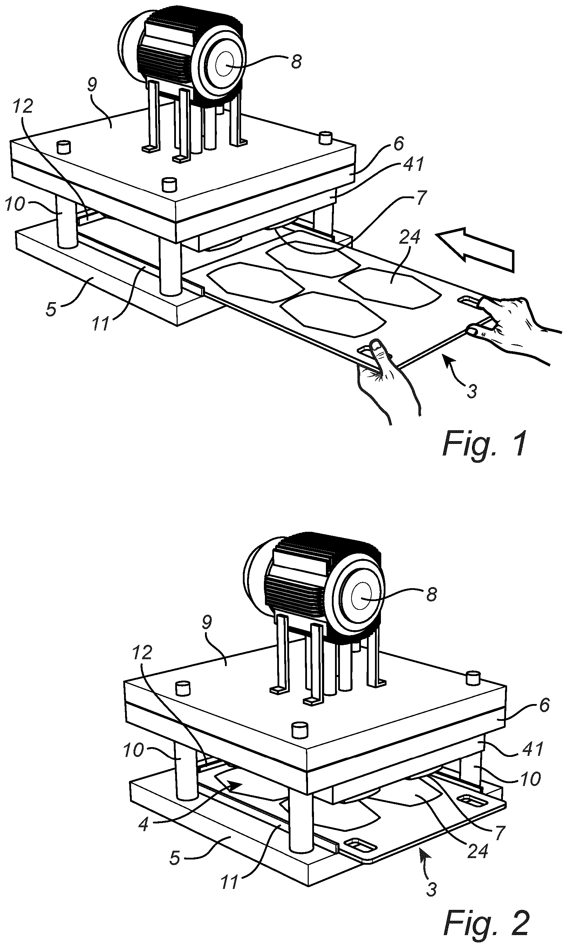

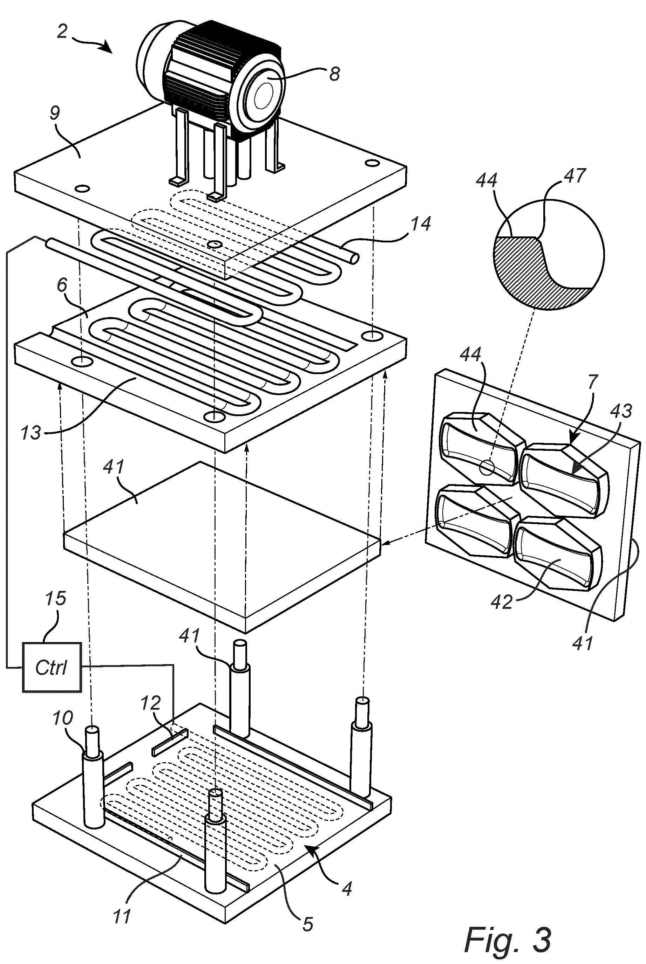

The arrangement 1 for manufacture of hygiene products in figure 1 and 2 generally includes a heat press 2 and a tray 3. The tray 3 is insertable into the heat press 2, more specifically into a space 4 formed between a baseplate 5 and a top plate 6 provided with a set of molds 7, here four molds. In the illustrated example, the baseplate 5 is stationary, while the top plate 6 with the molds 7 is movable in the vertical direction, between a first position, in which the space 4 is formed, and a second position in which the top plate 6 is moved downwards so as to be immediately above the baseplate 5. Turning to an exploded view of the heat press 2 in figure 3, an actuator 8 is operatively connected to the top plate, to effect displacement of the top plate 6 between the first and second position. In the illustrated example the actuator 8 is a hydraulic press, which has been configured to provide a suitable amount of pressure for manufacturing the hygiene products, e.g. in the order of one bar. The actuator 8 is connected to a frame 9 of the heat press 2. The frame 9 comprises four guide rails 10. The top plate 6 has four holes, one in each of the top plate's four corners. They are run through by the four guide rails 10, one through each hole. The guide rails 10 thereby serve to ensure that the top plate 6 remains parallel with the baseplate 5 while the top plate 6 moves.

The heat press 2, in the illustrated example the frame 9, further comprises guides 11, 12 that ensure that the tray is in a well-defined position when it is inserted into the space 4. The guides 11, 12 comprise two side guides 11, that ensure that the tray is inserted towards the specified position. The guides further comprise an inner abutment 12, that ensures that the tray is inserted the right depth into the heat press.

Both the baseplate 5 and the top plate 6 are configured to be heated to an elevated temperature. For this purpose, the baseplate 5 and top plate 6 are provided with grooves 13 in a meander pattern and electric heating cables 14 arranged in the grooves 13. The heating cables 14 are connected to a controller 15 that ensures a desired temperature of the plates 5, 6 by applying electric current to the heating cables 14. In order to ensure satisfactory heating, the baseplate 5 and the top plate 6 should be of a material with high thermal conductivity. In the illustrated example the plates 5, 6 are formed as aluminum slabs. Aluminum is also a material in which grooves 13 are easily formed.

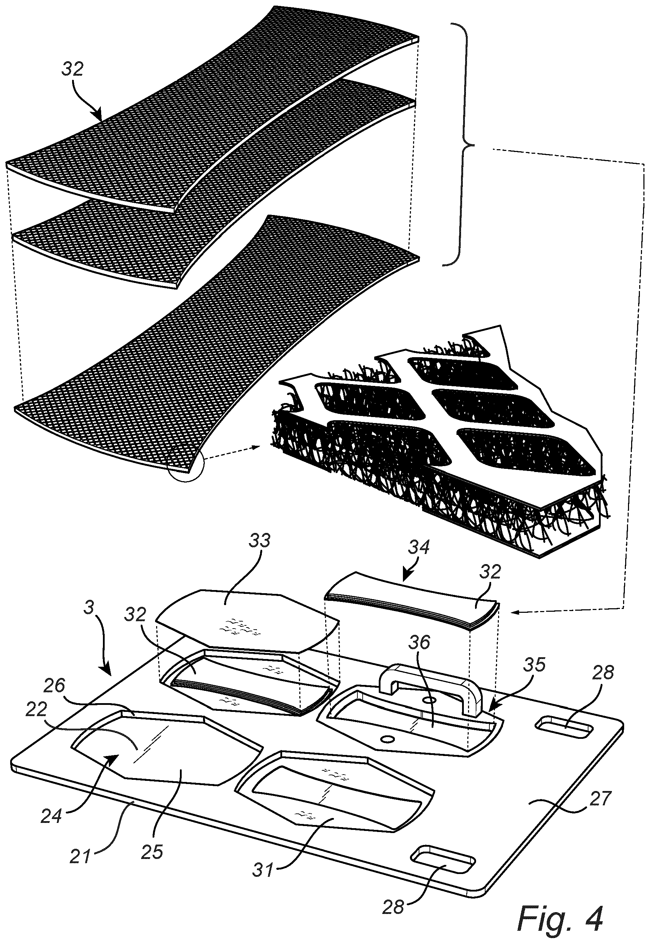

The tray 3 is shown in more detail in figure 4. The main part of the tray 3 is a slab 21 of a heat endurable material, such as Bakelite, MDF or plywood, which is formed with a set of at least one, and in the illustrated example four, holes 22. A heat transmissive film 23 is attached to the backside of the slab 21, so that each hole 22 forms a compartment 24 with a floor 25 formed by the film.

The film is preferably made of PTFE for avoiding adhesion of a heat sealable material to the bottom of a compartment 24 when heat is applied. For this reason, the walls 26 of the holes 22 are preferably also coated by PTFE film. For example, a self-adhesive PTFE tape may extend from a first side of the slab to a second side of the slab , through the compartment 22, fixing the tape along three surfaces, the first side of the slab 20a, the second side of the slab 20b and the walls 26 of the compartment.

In the illustrated example the slab 21 of the tray 3 further comprises a gripping section 27 for gripping and holding the tray. In the illustrated example, the gripping section has two through holes 28 allowing a user to grip and hold the tray 3, as is illustrated in figure 1. Other possible examples of gripping sections include a protruding handle, and easily grippable edges of the slab.

The compartment 24 is configured to receive a stack 30 of layers constituting material for making a washable hygiene product. In the illustrated example, the stack includes an annular liner 31 of a liquid impermeable heat sealable material, one or several patches 32 of a spacer material, and a back sheet 33 of a liquid impermeable heat sealable material.

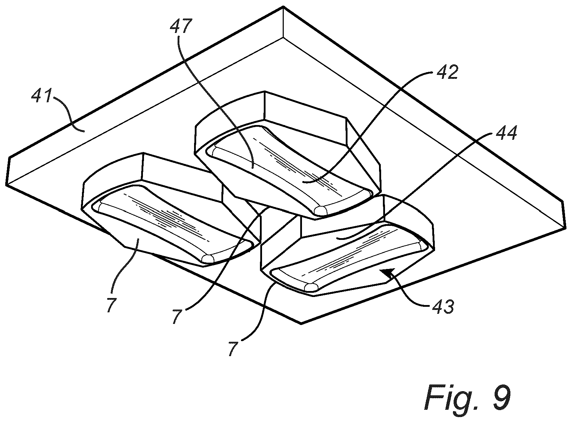

The patches 32 together form a liquid retaining pad 34, and may have slightly varying shape and thickness. The liner 31 and the back sheet 33 preferably have roughly the same size, while the patches 32 are smaller. In the illustrated example, the pad 34 includes two slightly larger patches 32 of spacer material and one slightly smaller patch 32. The outer contours of the annular liner 31 and the back sheet 33 are generally elongated with a wider center portion, and here have two rounded convex sides opposite each other, and two longer V-shaped convex sides connecting the two rounded convex sides. The contour of the pad 34, as defined by the lager patch 32, is generally elongated with a narrow central portion, and here has two convexly rounded sides opposite each other, and two longer concavely rounded sides connecting the two convexly rounded sides. As seen in figures 4 and 9, the outer contour of the compartment 24 matches the outer contour of the annular liner 31 and back sheet 33, so as to keep the stack of layers in place.

The arrangement 1 further comprises a compartment limiter 35 configured to be placed in a compartment 24 of the tray 3. The compartment limiter 35 should fit precisely into the compartment 24, while still allowing easy insertion and removal of the compartment limiter 35. The compartment limiter 35 comprises a central opening 36, having a shape snugly matching one of the larger patches 32.

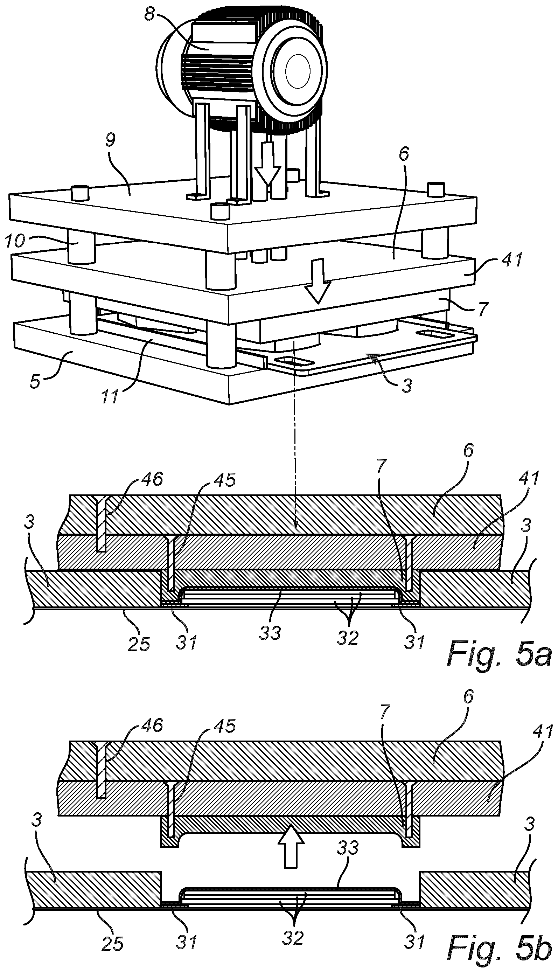

Returning to figures 1-3, the molds 7 may be made of a similar material as the plates 5 and 6, e.g. aluminum, and may be coated with an appropriate anti-adhesion coating, such as PTFE. The molds are here mounted on a carrier plate 41, e.g. by four screws 45 per mold 7 (see figure 5a-b). The four screws 45 are inserted through the carrier 41 plate into the mold 7, so that the face 43 of the mold 7 is unaffected by the four screws 45. The carrier plate 41 is in turn affixed to the top plate 6, here again by screws 46 (see figure 5a-b). The molds 7 are thermally connected to the top plate 6 via the carrier plate 41, ensuring the molds 7 are heated to the elevated temperature when the top plate 6 is heated to the elevated temperature. For the arrangement 1 to operate, the set of molds 7 in the heat press 2 needs to match the set of compartments 24 in the tray 3. An outer contour of each mold 7 therefore has the same shape as the outer contour of a corresponding compartment 24, in this case having two longer V-shaped sides, and two shorter curved sides.

Each mold 7 further comprises an indentation 42 on its face side 43, the indentation 42 having an outer contour corresponding to the shape of the larger patches 32 of the product it is designed to produce. A part of the face side 43 surrounding the indentation 42 is substantially flat and will be referred to as a flat part 44. A rim 47 of the flat part 44 immediately outside the indentation 42 is slightly inclined towards the indentation. The inclination is exaggerated in the figure, and may be as small as one degree. The rim 47 lowers maximal tensile stress in the annular liner 31 during heat pressing, by allowing a less sharp corner to form from adhering to the back sheet 33 and adhering to the liquid retaining patches 32. The rim 47 thereby reduces the risk of the liquid impermeable layers 31,33 tearing during heat pressing. The corner being less sharp also leads to a space forming inside of the corner allowing more liquid to be retained.

This shape is chosen since it is beneficial if the mold 7 is so shaped as to follow a topographical structure of the set of layers, with the mold having its flat part aligned with the area where the stack of layers is thinnest, while the indentation 42 caves in more where the set of layers is thicker, and less where the set of layers is thinner. This principle stems from the fact that spacer material of the patches 32 loses liquid retaining ability when pressed and heated, together with the fact that the patches 32 are smaller in area than the annular layer 31 and back sheet 33 and that the patches 32 are placed in a center of a product

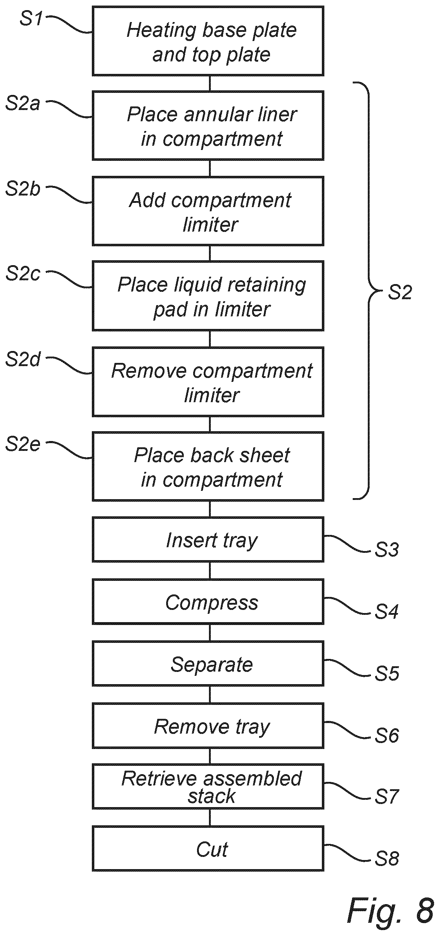

Operation of the arrangement 1 will now be described with reference to the flowchart in figure 8 and to figures 4-7.

First, in step S1 the controller 15 is operated, such that it sends an electric current to the heat cables 14 heating them up. Heat from the heat cables 14 then spreads from the heat cables 14 into the top plate 6 and the baseplate 5 heating them to the desired elevated temperature, whereafter heat is conducted into the carrier plate 41, and finally into the molds 7. This ensures that the baseplate 5 and the molds 7 are heated to the elevated temperature. The elevated temperature should be such, as to activate the adhesive properties of the heat sealable materials. In the currently described embodiment the elevated temperature is in the range 100 - 200 degrees Celsius, for example around 150 degrees C.

Then, in step S2, a stack 30 of layers 31, 32, 33 is arranged in each compartment of the tray. As illustrated in figure 4, the stacking step S2 begins with placing the annular layer 31 in the compartment 24 (step S2a), whereafter the compartment limiter 35 is inserted into the compartment 24 (step S2b). Then all patches 32, e.g. one, two or three, are placed in the compartment 24 in the hole of the compartment limiter 35 (step S2c). Then the compartment limiter 35 is removed (step S2d), whereafter the back sheet 33 is finally placed on top of the patches 32 (step S2e).

In the following step S3, the tray 3 is inserted into the space 4 between the top plate 6 and the base plate 5 between the side guides 11 until the tray 3 reaches the abutment 12. Inserted in this way the gripping section 27 protrudes out of the space 4 and each compartment 24 aligns with one mold 7 each.

Then, in step S4, the actuator 8 of the heat press 2 is operated whereby the top plate 6 is moved down towards the baseplate 5 so that each stack 30 is compressed between a respective mold 7 and the floor 25 of a respective compartment 24, as illustrated in figure 5a. The pressure applied by the actuator is chosen to activate the adhesive properties of the heat sealable materials, and in the illustrated example the actuator applies a pressure of 2 bar. At the same time, heat is applied to the stack 30 from the baseplate 5 through the heat transmissive floor 25 of the compartment 24 and from the top plate 6 via the mold 7. The indentation at the molds center leads to less pressure being applied there than at the mold's substantially flat part.

Step S4 is carried out for different number of seconds depending on the temperature of the pates 5, 6 and the specific type of heat sealable material used. As an example, S4 may be carried out for a duration of less than a minute, typically 15-30 seconds, for example around 20 seconds.

Then, in step S5, the actuator 8 is again operated to separate the top plate 6 from the baseplate 5, and the tray is then removed from the space 4 in step S6, as illustrated in figure 5b.

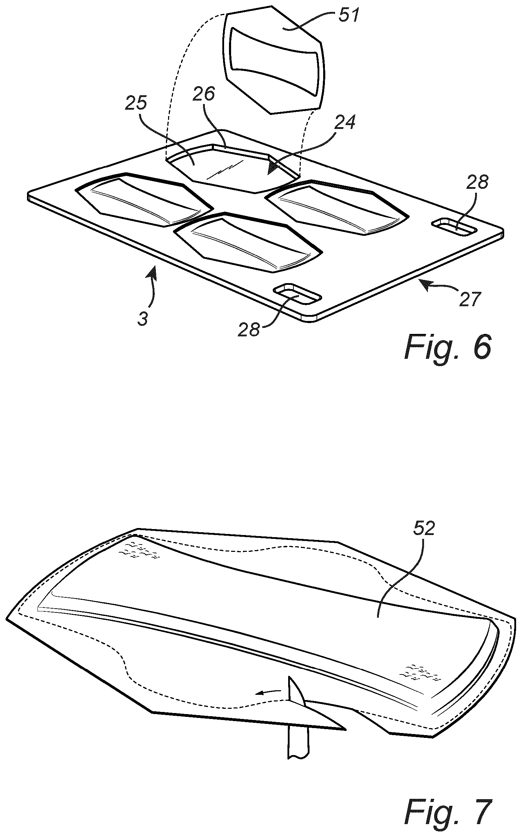

As shown in figure 6, in step S7 a now assembled stack 51 may now be retrieved from each compartment 24. Finally, in step S8, any surplus material is cut away from the assembled stack 51 and a fastening means such as a button or hook-and-loop material is added to help the reusable hygiene product maintain its position during use after which the reusable hygiene product 52 is ready to use.

The current invention is configured to produce products which comprise a stack of layers comprising: a liquid impermeable back sheet, a liquid retaining pad, and a liquid heat sealable impermeable annular liner. The skilled craftsperson will nevertheless realize that other products that have heat pressing as a step in their manufacture can have that step carried out with the arrangement disclosed herein.

The person skilled in the art realizes that the present invention by no means is limited to the preferred embodiments described above. On the contrary, many modifications and variations are possible within the scope of the appended claims. For example, the number of compartments and molds may be other than four. For a given size of the heated plates 5 and 6, the number of compartments/molds may vary depending on the desired size of the produced products. An alternative embodiment with three molds is shown in figure 9.

## Claims

1. An arrangement for producing a washable reusable hygiene product, the washable reusable hygiene product including of a stack of layers comprising: a liquid impermeable back sheet (33), a liquid retaining pad (34), and a heat sealable liquid impermeable annular liner (31),
**characterized in that** the arrangement comprises:
a baseplate (5), and
at least one mold (7) comprising a central indentation (42) facing the baseplate (5)
and a substantially flat section (44) surrounding the indentation (42), wherein the baseplate and at least one mold (7) are configured to be heated to an elevated temperature, and are displaceably arranged between a first position in which a space (4) is formed between the at least one mold (7) and the baseplate (5) and a second position in which the at least one mold (7) is in proximity with the base plate (5),
the arrangement further comprising a tray (3) arranged to be inserted in said space (4), said tray (3) comprising at least one compartment (24) having a heat transmissive floor (25) configured to support a stack (30) of layers arranged in the compartment (24), said tray being insertable into said space such that each mold (7) is aligned with a respective compartment (24),
so that, when the tray (3) is inserted into said space (4) and the at least one mold (7) and baseplate (5) are heated to the elevated temperature and displaced to the second position, each mold (7) is received by a respective compartment (24), thereby compressing a stack (30) of layers in the compartment (24) and heat sealing the annual liner (31) with the back sheet (33).

2. The arrangement according to claim (1), wherein each compartment (24) has a shape which snugly matches a respective mold (7).

3. The arrangement according to any of the previous claims wherein the indentation (42) in the mold (7) has a shape which follows the shape of the liquid retaining pad (34) of the product the arrangement is intended to produce.

4. The arrangement according to any of the previous claims, comprising two, three, or more, molds (7) and a corresponding number of compartments (24).

5. The arrangement according to any of the previous claims further comprising a compartment limiter (35), configured to be inserted into a compartment (24), wherein the compartment limiter (35) comprises a hole for receiving and ensuring proper placement of the liquid retaining pad (34) in the compartment (24).

6. The arrangement according to any of the previous claims, wherein the elevated temperature is between 130-170 degrees Celsius, preferably between 140 and 160 degrees Celsius.

7. The arrangement according to any of the previous claims, wherein the tray (2) further comprises a PTFE film which is attached to one side of the tray (3) to form the heat transmissive floor (25) of the tray (3).

8. The arrangement according to any of the previous claims, wherein inwards-facing walls (26) of the compartment (24) are covered substantially by PTFE tape.

9. The arrangement according to any of the previous claims, further comprising an actuator (8) connected to a frame (9), wherein the actuator configured to displace the base plate (5) and at least one mold (7) between the first and second position, and wherein the at least one mold (7) are situated on a top plate (6) connected to the actuator (8).

10. The arrangement according to any of the previous claims, wherein the mold (7) is mounted on a top plate (6) with thermal connection, wherein the top plate comprises a first groove (13) a first heat cable (14) placed in the first groove (13), and
wherein the baseplate (5) comprises a second groove (13) a second heat cable (14) placed in the second groove (13)
wherein the first heat cable (14) and the second heat cable (14) are connected to a controller (15) which controls a heat output of the heat cables (14) to the top plate (6) and to the baseplate (5),
wherein the heat output heats the base plate (5) and the top plate (6) to the elevated temperature.

11. The arrangement according to any of the previous claim, wherein the liquid retaining pad (34) is made from spacer material.

12. The arrangement according to any of the previous claims, further comprising a set of guides (11,12) that ensure that the tray is inserted into a position where each mold (7) is aligned with a respective compartment (24).

13. A method for producing a washable reusable hygiene product comprising the steps:
- providing a tray (3) with a compartment (24) and a heat transmissive floor (25),
- arranging a stack (30) of layers in the compartment (24) (S2), wherein the stack (30) includes a liquid impermeable annular liner (31), a liquid retaining pad (34), and a liquid impermeable back sheet (33):
- inserting the tray (3) into a space (4) between a heated baseplate (5) and a heated mold (7),
- displacing the mold (7) in relation to the baseplate (5) so that they press the stack (30) from opposite directions, thereby heat-sealing the annular liner and the liquid retaining pad to the back sheet (33), to form an assembled stack (51), and
- retrieving the assembled stack (51) from the compartment.

14. The method according to claim 13, wherein the assembling step (S2) further comprises the steps of:
- adding (S2b) a compartment limiter (35) to the compartment (24) before arranging (S2c) the liquid retaining pad (34),
- removing (S2d) the compartment limiter (35) after arranging the liquid retaining pad (34),
wherein the arranging of the liquid retaining pad (34) is guided by the compartment limiter (35).

15. The method according to claim 13 or 14, wherein the liquid retaining pad comprises a smaller patch facing the back sheet, a larger patch facing the liner, and at least one intermediate patch, sandwiched between the smaller and larger patches, said intermediate patch being larger than the smaller patch.
